# EUROPEAN PATENT APPLICATION

(11) **EP 2 420 487 A1**
(43) Date of publication of application: **22.02.2012**
(21) Application number: 10764432.0
(22) Date of filing: 13.04.2010
(51) Int. Cl.: C07C 253/30, C07B 53/00, C07C 255/24, C07D 207/16, C07B 61/00

(54) **NOVEL METHOD FOR PRODUCING OPTICALLY ACTIVE PYRROLIDINE COMPOUND**

(30) Priority: 14.04.2009 JP 2009097542
(71) Applicant: Astellas Pharma Inc., Tokyo 103-8411 (JP)
(72) Inventor: OHIGASHI, Atsushi, Tokyo 103-8411 (JP); KIKUCHI, Takashi, Tokyo 103-8411 (JP)
(74) Representative: Gille Hrabal
(86) International application number: PCT/JP2010/056549
(87) International publication number: WO 2010/119848

(57) **Abstract**

[Object]

A novel method for producing an optically active pyrrolidine compound, which is useful as a production intermediate of a pharmaceutical, and a production intermediate thereof, is provided.

[Means for Solution]

According to the production method of the present invention, a chloro compound that is a key intermediate can be produced efficiently industrially by subjecting a mixture of regioisomers obtained by reacting an optically active epoxy compound substituted with aryl, which is easily available, with an amine compound, to chlorination. Furthermore, an optically active pyrrolidine compound can be produced industrially efficiently with the key intermediate.

## Description

### Technical Field

The present invention relates to a novel method for producing an optically active pyrrolidine compound, which is useful as a starting material for producing a pharmaceutical, and an intermediate thereof.

### Background Art

Optically active pyrrolidine compounds represented by the following formulae (7) and (8) that can be produced by the production method of the present invention are useful as a starting material for production of a pharmaceutical. 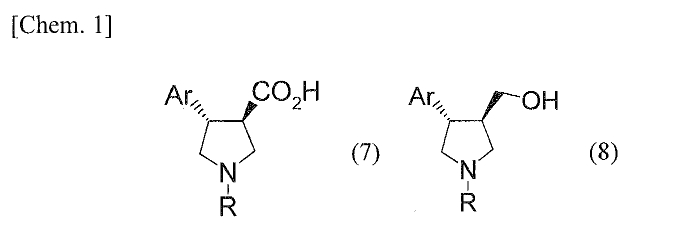 (In the formula, Ar represents aryl which may be substituted and R represents lower alkyl or lower alkylene-aryl. In this regard, the aryl in R may be substituted.)

For example, it is described that a compound represented by the following formula (A) is useful as a calcium-sensing receptor agonist in Patent Document 1, and the compounds represented by the formula (7) and formula (8) can be used as a starting material for production of the compound represented by the formula (A). 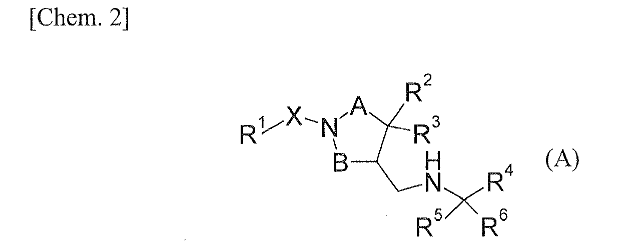 (The symbols in the formula represent the following meanings.
A and B: -C(R⁷)(R^{7a})- or -C(O)-.
R⁷ and R^{7a}: -H, lower alkyl, aryl, or the like.
R² and R³: -H, lower alkyl, halogeno-lower alkyl, -OC(O)-R⁰, cycloalkyl, lower alkylene-cycloalkyl, aryl, or the like.
R⁰: -H or lower alkyl.
Refer to the publication for the other symbols.)

As a method for producing the optically active pyrrolidine compounds represented by the formula (7) or formula (8), the methods as in Patent Documents 1 to 4 are reported.
In Patent Document 1, there is described a production method represented by the following production method A. In the production method A, in the presence of trifluoroacetic acid, azomethine ylide formed from N-benzyl-1-methoxy-N-[(trimethylsilyl)methyl]methanamine and an acrylic acid derivative (A1) in which Y is an asymmetric auxiliary group are subjected to a cycloaddition reaction, and then to reduction, thereby producing an optically active pyrrolidine compound (A3).

### (Production Method A)

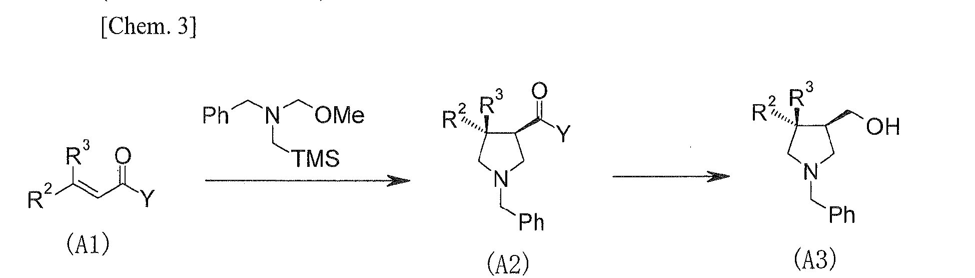

(In the formula, R² and R³ have the same meanings as in the formula (A). The other symbols represent the following meanings.
Y: asymmetric auxiliary groups such as (S)-4-benzyl-2-oxazolidinone and the like, Me: methyl, TMS: trimethylsilyl, and Ph: phenyl.)

In Patent Document 2, there is described a production method represented by the following production method B. In the production method B, in the presence of trifluoroacetic acid, azomethine ylide formed from N-benzyl-1-methoxy-N-[(trimethylsilyl)methyl]methanamine and an acrylic acid compound (B1) having an asymmetric auxiliary group are subjected to a cycloaddition reaction to obtain a compound (B3) as a main product, which is then subjected to hydrolysis, thereby producing an optically active pyrrolidine compound (B4).

### (Production Method B)

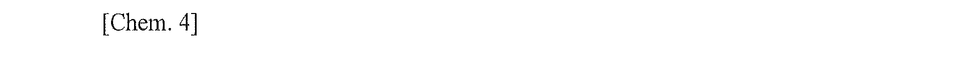

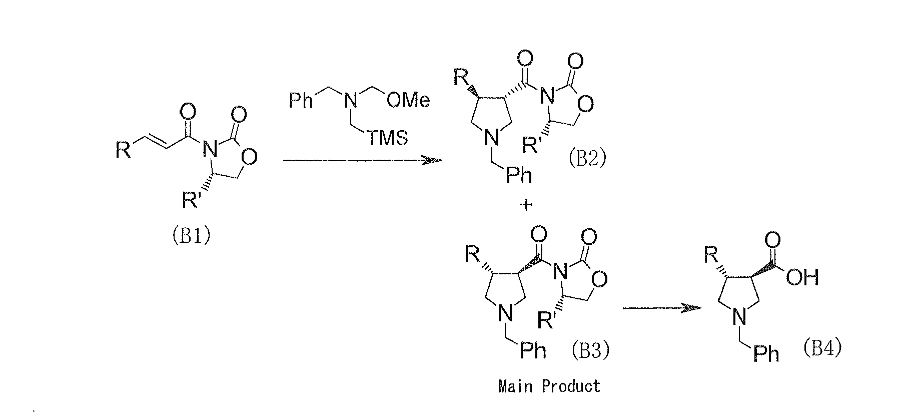

(The symbols in the formula have the following meanings.
R: C₁-C₆ alkyl, C₃-C₅ cycloalkyl, phenyl, or the like, Me: methyl, TMS: trimethylsilyl, Ph: phenyl, and R': isopropyl, isobutyl, t-butyl, phenyl, benzyl, or the like.)

In Patent Document 3, there is described a production method represented by the following production method C. In the production method C, in the presence of trifluoroacetic acid, azomethine ylide formed from N-benzyl-1-methoxy-N-[(trimethylsilyl)methyl]methanamine and an acrylic acid compound (C1) having an asymmetric auxiliary group are subjected to a cycloaddition reaction, and then to reduction, thereby producing optically active pyrrolidine compounds (C4) and (C5).

### (Production Method C)

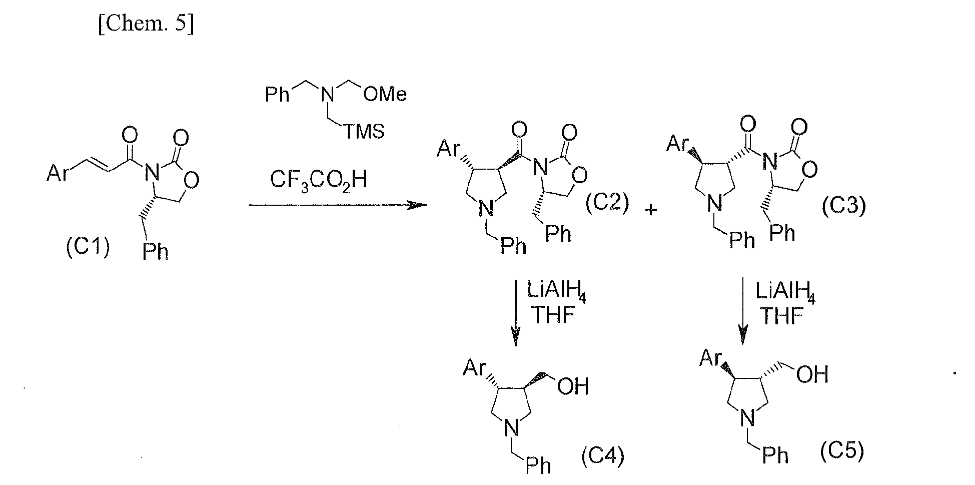

(The symbols in the formula represent the following meanings.
Ar: phenyl, naphthyl, heterocycle group, Me: methyl, TMS: trimethylsilyl, Ph: phenyl, and THF: tetrahydrofuran.)

In Patent Document 4, there is described a production method represented by the following production method D. In the production method D, a compound (D2) obtained by asymmetric reduction of a compound (D1) and an amine compound (D3) are reacted and then subjected to a Michael addition to obtain a compound (D7), which is then subjected to cyclization and hydrolysis, thereby producing an optically active pyrrolidine compound (D10).

### (Production Method D)

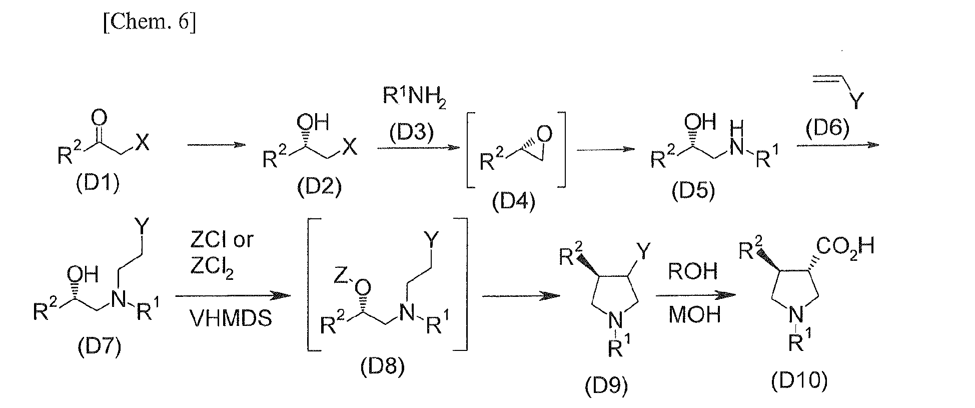

(The symbols in the formula represent the following meanings.
R¹: hydrogen, amidino, C₁₋₄ alkyliminoyl, C₁₋₁₀ alkyl, or the like,
R²: C₁₋₄ alkyl, -(CH₂)ₙ-cycloalkyl, -(CH₂)ₙ-heteroeycloalkyl, -(CH₂)ₙ-heterophenyl, or the like,
n: 0, 1, 2, 3, or 4,
X: bromo or chloro,
Y: -CN or -CO₂R⁵,
Z: -PO(OR⁶)₂, -PO(N(R⁶)₂)₂, methanesulfonyl, or p-toluenesulfonyl,
V: alkali metal, and
HMDS: hexamethyl disilazane.
Refer to the publication for the other symbols.)

In Non-Patent Document 1, there is described a production method represented by the following production method E. In the production method E, a chloro compound (E2) obtained from a phenacyl bromide compound (E1) is subjected to cyclization using a base, and then to ethanolysis and hydrolysis, thereby producing a racemic pyrrolidine compound (E5). Furthermore, there is also described a method in which a compound (E7) that is separately produced is subjected to chlorination together with rearrangement, thereby producing a chloro compound (E2). However, a method for producing optically active forms of the pyrrolidine compound (E5) and the chloro compound (E2) is not described.

### (Production Method E)

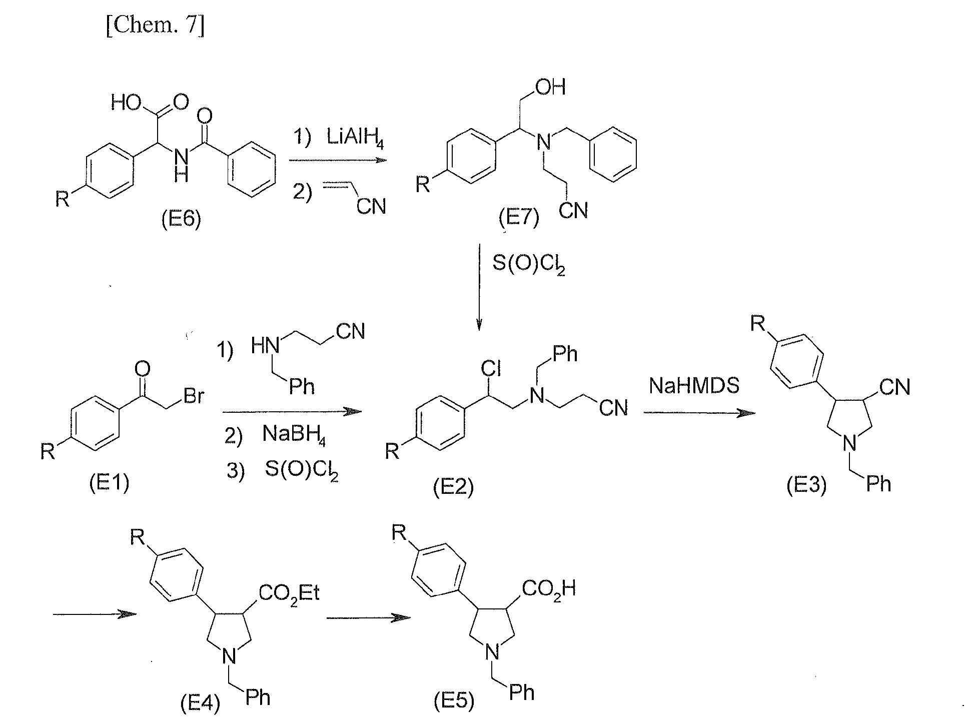

(The symbols in the formula represent the following meanings.
R: -H, -Cl, -F, or methyl, Ph: phenyl, Et: ethyl, and NaHMDS: sodium hexamethyldisilazide).

### List of the Documents

### Patent Documents

Patent Document 1: Pamphlet of International Publication WO 2006/123725
Patent Document 2: Specification of U.S. Patent No. 5618949
Patent Document 3: Pamphlet of International Publication WO 2000/59502
Patent Document 4: Pamphlet of International Publication WO 2004/092126

### Non-Patent Document

Non-Patent Document 1: Achini, et al., "Helvetica Chimica Acta", 1981, Vol. 64, p. 2203-2218

### Summary of the Invention

### Problems that the Invention is to Solve

Conventional methods for producing the optically active pyrrolidine compounds (7) and (8) have not been satisfactory industrially. Thus, there is a demand for development of methods for efficiently producing the optically active pyrrolidine compounds (7) and (8).

### Means for Solving the Problems

The present inventors have extensively studied so as to develop the optically active pyrrolidine compounds (7) and (8) that are more efficient industrially. As a result, the present inventors have found that the optically active pyrrolidine compounds (7) and (8), and a production intermediate thereof are produced efficiently by the following production method, thereby completing the present invention.
The present invention relates to a novel method for producing the optically active pyrrolidine compounds (7) and (8) and a production intermediate thereof. That is:
[1] A method for producing a compound represented by the formula (3) 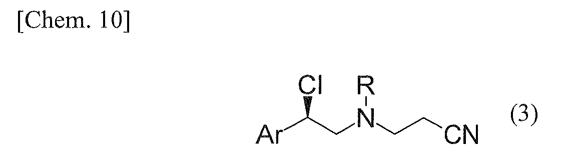 [wherein Ar and R have the same meanings as in the formula (1)] or a salt thereof, which comprises reacting
   a compound represented by the formula (1) 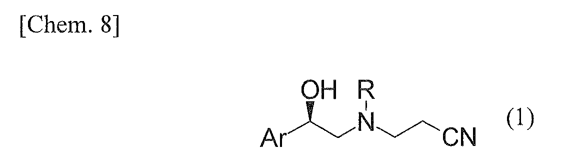 [wherein Ar represents aryl which may be substituted and R represents lower alkyl or lower alkylene-aryl, provided that the aryl in R may be substituted] or a salt thereof, or
   a compound represented by the formula (2) 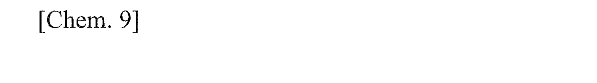 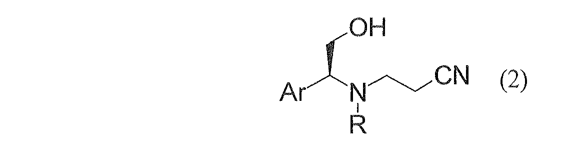 [wherein Ar and R have the same meanings as in the formula (1)] or a salt thereof, or
   a mixture of the compound represented by the formula (1) or a salt thereof and the compound represented by the formula (2) or a salt thereof,
   with a compound represented by lower alkyl-S(O)₂Cl, in the presence of a base.
[2] The production method as described in [1],
   wherein the compound represented by the formula (1) or a salt thereof or the compound represented by the formula (2) or a salt thereof, or a mixture of the compound represented by the formula (1) or a salt thereof and the compound represented by the formula (2) or a salt thereof, which is produced by reacting a compound represented by the formula (4) 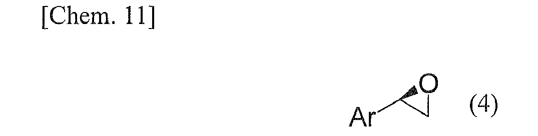 [wherein Ar has the same meaning as in [1]] with a compound represented by the formula (5) 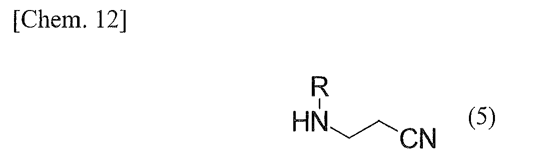 [wherein R has the same meaning as in [1]] or a salt thereof,
   is used as a starting material.
[3] A method for producing a compound represented by the formula (6) or a salt thereof, which comprises subjecting the compound represented by the formula (3) or a salt thereof produced by the production method as described in [1] or [2] to cyclization using a base: 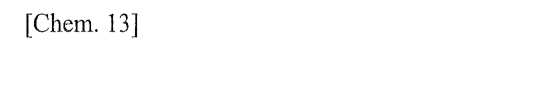 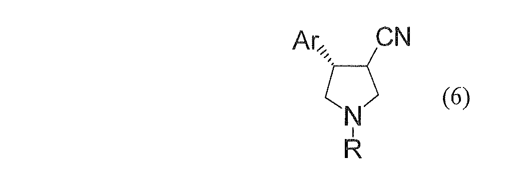 [wherein Ar and R have the same meanings as in [1]].
[4] A method for producing a compound represented by the formula (7) or a salt thereof, which comprises subjecting the compound represented by the formula (6) or a salt thereof produced by the production method as described in [3] to hydrolysis using a base: 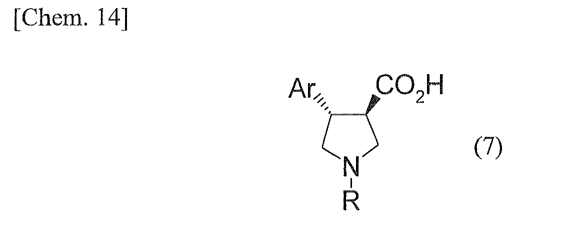 [wherein Ar and R have the same meanings as in [1]].
[5] A method for producing a compound represented by the formula (8) or a salt thereof, which comprises subjecting the compound represented by the formula (7) or a salt thereof produced by the production method as described in [4] to reduction: 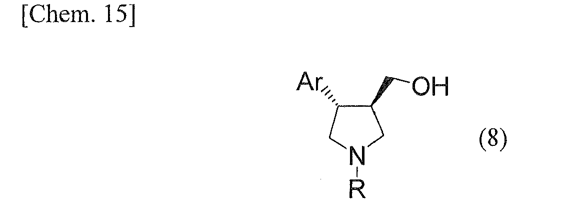 [wherein Ar and R have the same meanings as in [1]].
[6] The production method as described in [1], wherein Ar is phenyl which may be substituted.
[7] The production method as described in [1], wherein R is -CH₂-(phenyl which may be substituted).
[8] The production method as described in [2], wherein, in the step of reacting the compound represented by the formula (4) or a salt thereof with the compound represented by the formula (5) or a salt thereof in [2], the compound represented by the formula (4) or a salt thereof and the compound represented by the formula (5) or a salt thereof are reacted in the presence of phenol.

### Effects of the Invention

As a method for producing an optically active pyrrolidine compound, the above-described production methods A to D are reported.
In all of the production methods A to C, in the presence of trifluoromethanesulfonic acid, azomethine ylide formed from N-benzyl-1-methoxy-N-[(trimethylsilyl)methyl]methanamine and an acrylic acid compound having an asymmetric auxiliary group are subjected to a cycloaddtion reaction, thereby producing an optically active pyrrolidine compound. However, N-benzyl-1-methoxy-N-[(trimethylsilyl)methyl]methanamine is not readily available in large amounts, and in addition, the diastereoselectivity of the cycloaddition reaction is not sufficient and a silica gel column is used to remove minor isomers, which is thus not appropriate for industrial production.

In the production method D, the compound (D2) obtained by asymmetric reduction of the compound (D1) and the amine compound (D3) are reacted, and then subjected to a Michael addition to obtain the compound (D7), which is then subjected to cyclization and hydrolysis, thereby producing the optically active pyrrolidine compound (D10). However, in the addition reaction of the amine compound (D3), a large excess of the primary amine compound (D3) is used for control of regioselectivity in order to control the regioselectivity in the ring-opening of the intermediate epoxy compound (D4), and further, a large excess of the compound (D6) is used in the Michael addition reaction, and thus it is not efficient industrially.
However, according to the production method of the present invention, the optically active epoxy compound (4) substituted with aryl, that is easily available at low cost, and the amine compound (5) are reacted, and the obtained regioisomer(s) (1) and/or (2) is/are subjected to chlorination, whereby the same optically active chloro compounds (3) can be produced from both regioisomers. Accordingly, it is not necessary to control the regioselectivity of the reaction between the optically active epoxy compound (4) substituted with aryl and the amine compound (5), and further, the regioisomers (1) and (2) can be used as a mixture without isolation. Therefore, the optically active chloro compound (3) can be produced industrially efficiently.
Further, by using the obtained optically active chloro compound (3) as a key intermediate, the optically active pyrrolidine compounds (7) and (8) can be produced industrially efficiently.

Moreover, in the production method E, there is reported a method for producing the racemic pyrrolidine compound (E5), but in the production of an optically active form by optical resolution of racemates, there is a need for extra starting materials for the production of unnecessary optical isomers, which is thus not efficient industrially. However, according to the production method, the optically active epoxy compound substituted with aryl, that is a starting material, is readily available. In addition, in the production method E, the racemic chloro compound (E2) is produced through three steps, but according to the present production method, the optically active chloro compound (3) that corresponds to the compound (E2) can be produced through two steps, and the number of steps can be reduced, which is thus efficient industrially.

As described above, according to the production method of the present invention, the optically active pyrrolidine compounds (7) and (8), and a production intermediate thereof, particularly, the optically active chloro compound (3) which is a key intermediate can be produced efficiently.

### Mode for Carrying out the Invention

The present invention is further described as follows.
In the present specification, the "lower alkyl" is preferably linear or branched alkyl having 1 to 6 carbon atoms (which is hereinafter simply referred to as C₁₋₆), and specifically, a methyl group, an ethyl group, an n-propyl group, an isopropyl group, an n-butyl group, an isobutyl group, a sec-butyl group, a tert-butyl group, an n-pentyl group, an n-hexyl group, or the like. It is more preferably C₁₋₄ alkyl, and still more preferably methyl, ethyl, n-propyl, or isopropyl.

The "lower alkylene" is preferably linear or branched C₁₋₆ alkylene, and specifically, a methylene group, an ethylene group, a trimethylene group, a tetramethylene group, a pentamethylene group, a hexamethylene group, a propylene group, a methylmethylene group, an ethylethylene group, a 1,2-dimethylethylene group, or a 1,1,2,2-tetramethylethylene group or the like. It is more preferably C₁₋₄ alkylene, still more preferably methylene, ethylene, or trimethylene, and even still more preferably methylene.

The "halogen" means F, Cl, Br, or 1.
The "aryl" refers to a C₆₋₁₄ monocyclic to tricyclic aromatic hydrocarbon ring group, more preferably phenyl or naphthyl, and still more preferably phenyl.

The expression "which may be substituted" represents "which is not substituted" or "which is substituted with 1 to 5 substituents, which may be the same as or different from one other".

The substituent that is acceptable in the "aryl" which may be substituted in Ar and the "aryl" which may be substituted in R is preferably lower alkyl, halogen, or -O-lower alkyl.

The compounds represented by the formula (1) to formula (4) and the formula (6) to formula (8) are described in the form of any one optical isomer, but by using an optical isomer having an opposite configuration as a starting material, it is possible to produce an optical isomer having an opposite configuration. In the present invention, the production methods in which all the configurations of the formula (1) to formula (4) and the formula (6) to formula (8) are opposite are also included.

The starting material compounds and the products in the present production method may form an acid addition salt or a salt with a base depending on the kind of substituents. Specific examples thereof include acid addition salts with inorganic acids such as hydrochloric acid, hydrobromic acid, hydroiodic acid, sulfuric acid, nitric acid, and the like, and with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid, and the like, and salts with inorganic bases such as sodium, potassium, magnesium, calcium, aluminum, and the like, or salts with organic bases such as methylamine, ethylamine, ethanolamine, lysine, ornithine, and the like, ammonium salts, etc.

Ar is preferably phenyl which may be substituted, more preferably phenyl which may be substituted with halogen, and still more preferably unsubstituted phenyl.
R is preferably -CH₂-(phenyl which may be substituted), and more preferably unsubstituted benzyl.

The "base" that is used for chlorination of the compounds represented by the formula (1) and formula (2) is not particularly limited as long as it is a base usable in the reaction, but it is preferably triethylamine or diisopropylethylamine, and more preferably triethylamine.
The "base" that is used for cyclization of the compound represented by the formula (3) is not particularly limited as long as it is a base usable in the reaction, but it is preferably lithium hexamethyldisilazide, sodium hexamethyldisilazide, or potassium hexamethyldisilazide.
The "base" that is used for hydrolysis of the compound represented by the formula (6) is not particularly limited as long as it is a base usable in the reaction, but it is preferably lithium hydroxide, sodium hydroxide, potassium hydroxide, or calcium hydroxide, and more preferably sodium hydroxide.
The reducing agent that is used for reduction of the compound represented by the formula (7) is not particularly limited as long as it is a reducing agent usable in the reaction, but it is preferably borane, lithium aluminum hydride, or sodium bis(2-methoxyethoxy)aluminum hydride (registered trademark: Red-Al), and more preferably sodium bis(2-methoxyethoxy)aluminum hydride.

The production method of the present invention is presented below and each of the steps will be further described.

### (Production Method)

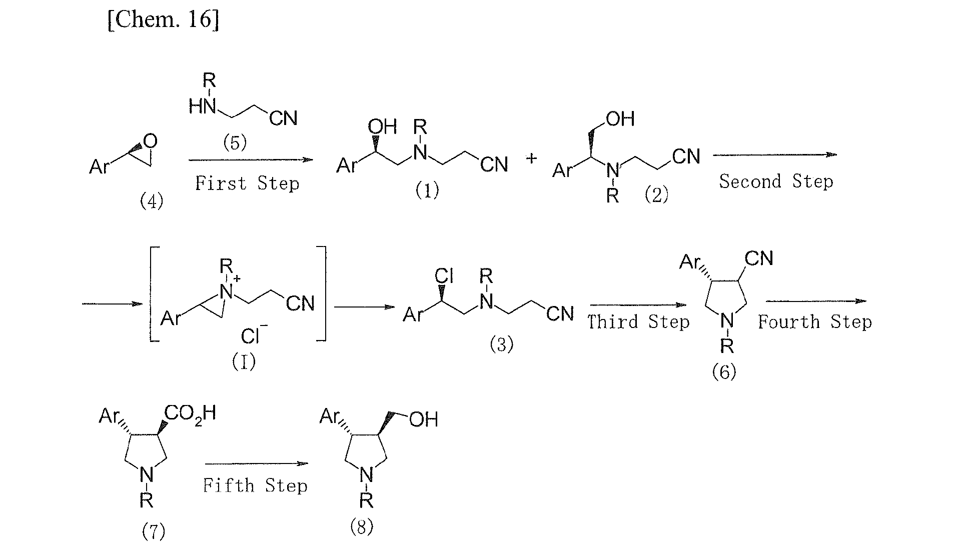

### First Step

The present step is a step in which an optically active compound (4) is subjected to a ring-opening reaction with a compound (5) to produce a compound (1) and a compound (2).
The ring-opening reaction can be carried out under from room temperature to heating, using the compound (4) and the compound (5) in equivalent amounts or with either thereof in an excess amount. The solvent is not particularly limited as long as it does not adversely influence the reaction, but as the solvent, alcohols such as methanol, ethanol, n-propanol, 2-propanol, and the like are preferably used. It may be advantageous in some cases for the progress of the reaction to carry out the reaction in the presence of Lewis acids such as zinc iodide and the like or Bronsted acids such as acetic acid, phenol, and the like (preferably phenol), depending on the compounds. The compound (1) and the compound (2) are usually obtained as a mixture, but they may be used in the next step as being respectively isolated or as a mixture.

### Second Step

The present step is a step in which the compound (1) or the compound (2), or a mixture of the compound (1) and the compound (2) is subjected to chlorination to produce a compound (3).
In the chlorination reaction of the present step, by using either the compound (1) or the compound (2) as a starting material, the reaction progresses via an aziridinium salt (I) as a common intermediate, and the same compound (3) can be obtained. Accordingly, the compound (1) and the compound (2) may be used as the starting materials in the form of a mixture.
The chlorination reaction can be carried out under from cooling to heating, using lower alkyl-S(O)₂Cl (preferably methanesulfonyl chloride) as a chlorinating agent in an equivalent amount or an excess amount relative to the compound (1) or the compound (2), or a mixture of the compound (1) and the compound (2), in the presence of a base. As the base, organic bases such as triethylamine, diisopropylethylamine, and the like can be used. The solvent is not particularly limited as long as it does not adversely influence the reaction, but aromatic hydrocarbons such as benzene, toluene, xylene, and the like are preferably used.

### Third Step

The present step is a step in which the compound (3) is subjected to cyclization in the presence of a base to produce a compound (6).
The cyclization reaction can be carried out under cooling in the presence of a base in an equivalent amount or an excess amount relative to the compound (3). As the base, lithium hexamethyldisilazide, sodium hexamethyldisilazide, potassium hexamethyldisilazide, or the like can be used. The solvent is not particularly limited as long as it does not adversely influence the reaction, but ethers such as diethylether, tetrahydrofuran, 1,2-dimethoxyethane, and the like, or aromatic hydrocarbons such as toluene, benzene, and the like are preferably used.
The compound (6) is usually obtained as a mixture of cis isomer and trans isomer, but it may be used in the form of a mixture in the next step.

### Fourth Step

The present step is a step in which the compound (6) is subjected to hydrolysis/isomerization in the presence of a base to produce a compound (7).
The hydrolysis/isomerization reaction can be carried out under from room temperature to heating in the presence of a base in an equivalent amount or an excess amount relative to the compound (6). As the base, lithium hydroxide, sodium hydroxide, potassium hydroxide, calcium hydroxide, or the like can be used. The solvent is not particularly limited as long as it does not adversely influence the reaction, but alcohols or aqueous solvents thereof are suitably used. Even in the case of using the compound (6) that is a mixture of cis isomer and trans isomer as a starting material, the cis isomer can be converted to the trans isomer to selectively obtain the compound (7) that is a trans isomer.

### Fifth Step

The present step is a step in which the compound (7) is subjected to reduction to produce a compound (8).
The reduction reaction can be carried out under from cooling to heating, using a reducing agent in an equivalent amount or an excess amount relative to the compound (7). As the reducing agent, borane, lithium aluminum hydride, sodium bis(2-methoxyethoxy)aluminum hydride, or the like can be used. The solvent is not particularly limited as long as it does not adversely influence the reaction, but aromatic hydrocarbons and ethers are suitably used.

Further, the starting material compound (2) can also be produced by the following method.

### (Starting Material Synthesis)

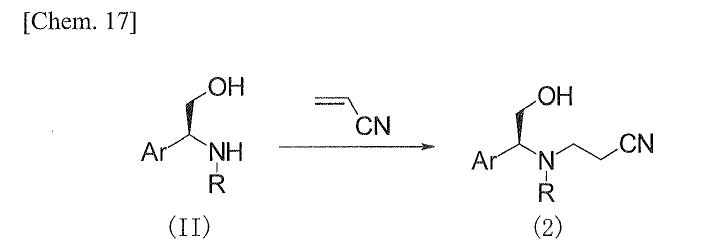

The present production method is a method in which a compound (II) and acrylonitrile are subjected to an addition reaction to obtain the compound (2).
The addition reaction can be carried out under heating, using acrylonitrile in an equivalent amount or an excess amount relative to the compound (II). The solvent is not particularly limited as long as it does not adversely influence the reaction, but ethers, aromatic hydrocarbons, or acrylonitriles can be used as a solvent. It may be advantageous in some cases for the progress of the reaction to carry out the reaction in the presence of an acid such as acetic acid and the like, depending on the compounds.

### Examples

The present invention will be specifically described with reference to Examples below, but the present invention is by no means limited to these Examples.

### Example 1

(1) To a four-neck flask which had been sufficiently substituted with nitrogen were put 300 g (2.50 mol, 99.0%ee or more) of (R)-(+)-styrene oxide, 480 g (3.00 mol) of 3-(benzylamino)propionitrile, 235 g (2.50 mol) of phenol, and 300 mL of ethanol, followed by stirring at 81 to 83°C for 8 hours. After cooling to 10°C, 850 mL of toluene and 1.50 L of water were added thereto. After adjusting to pH 4.0 with 52 mL of concentrated hydrochloric acid, the organic layer (the upper layer) was collected by separation and washed with 600 mL of tap water. To the organic layer (the upper layer) was added 1.50 L of water, followed by adjusting to pH 12 with 208 mL of a 24% aqueous sodium hydroxide solution, and the organic layer (the upper layer) was collected by separation. To the organic layer was added 1.50 L of water, followed by adjusting to pH 12 with 34 mL of a 24% aqueous sodium hydroxide solution, and the organic layer (the upper layer) was collected by separation. To the organic layer was added 1.50 L of water, followed by adjusting to pH 12 with 46 mL of a 24% aqueous sodium hydroxide solution. The organic layer collected by separation was washed sequentially with 900 mL of water and brine (180 g of salt/720 mL of water). The obtained organic layer was concentrated under reduced pressure, and to the residue was added 600 mL of toluene. After concentrating under reduced pressure again, 791 g of a crude product (oily substance) of a mixture (69:31) of 3-{benzyl[(2R)-2-hydroxy-2-phenylethyl]amino}propanenitrile and 3-{benzyl[1S)-2-hydroxy-1-phenylethyl]amino}propanenitrile was obtained.
   In this regard, the mixture of 3-{benzyl[(2R)-2-hydroxy-2-phenylethyl] amino }propanenitrile and 3-{benzyl[(1S)-2-hydroxy-1-phenylethyl]amino}propanenitrile was separated and purified by silica gel column chromatography, and each of the structures was confirmed.
   3-{Benzyl[(2R)-2-hydroxy-2-phenylethyl]amino}propanenitrile
   ¹H NMR (CDCl₃, 400 MHz) δ (ppm) 7.39-7.24 (m, 10H), 4.76-4.69 (m, 1H), 3.92 (d, J=13.6, 1H), 3.62 (d, J=13.6, 1H), 3.47 (d, J=0.8, 1H), 3.02-2.95 (m, 1H), 2.85-2.75 (m, 2H), 2.66-2.60 (m, 1H), 2.47-2.43 (m, 2H).
   3-{Benzyl[(1S)-2-hydroxy-1-phenylethyl]amino}propanenitrile
   ¹H NMR (CDCl₃, 400 MHz) δ (ppm) 7.42-7.23 (m, 10H), 4.14-4.07 (m, 1H), 3.96-3.92 (m, 1H), 3.86 (d, J=13.6, 1H), 3.75-3.69 (m, 1H), 3.38 (d, J=13.6, 1H), 3.09-3.02 (m, 1H), 2.70 (dd, J=2.0, 9.2, 1H), 2.63-2.57 (m, 1H), 2.46-2.23 (m, 2H)
(2) To a four-neck flask that had been sufficiently substituted with nitrogen were put 788 g of a crude product of a mixture of 3-{benzyl[(2R)-2-hydroxy-2-phenylethyl]amino}propanenitrile and 3-{benzyl[(1S)-2-hydroxy-1-phenylethyl]amino}propanenitrile, 2.79 L of toluene, and 352 g (3.49 mol) of triethylamine. At 6 to 15°C, 342 g (2.99 mol) of methanesulfonyl chloride was added dropwise thereto, followed by stirring at 20 to 23°C for 3 hours. To the reaction liquid was added 2.09 L of water, and the organic layer (the upper layer) was collected by separation. The organic layer collected by separation was washed sequentially with an aqueous sodium hydrogen carbonate solution (105 g of sodium hydrogen carbonate/2.10 L of water) and brine (420 g of salt/2.10 L of water). The obtained organic layer was concentrated under reduced pressure, and to the residue was added 1.40 L of toluene. After concentrating under reduced pressure again, 1239 g of a crude product (oily substance) of 3-{benzyl[(2R)-2-chloro-2-phenylethyl]amino}propanenitrile was obtained.
   In this regard, the obtained 3-{benzyl[(2R)-2-chloro-2-phenylethyl]amino}propanenitrile was purified by silica gel column chromatography, and the structure was confirmed.
   ¹H NMR (CDCl₃, 400 MHz) δ (ppm) 7.37-7.23 (m, 10H). 4.80 (t, J=6.8, 1H), 3.74 (d, J=13.6, 1H), 3.68 (d, J=13.6, 1H), 3.21-3.08 (m, 2H), 2.85 (t, J=6.8, 2H), 2.30-2.25 (m, 2H)
   MS (API-ES (Pos.)) (m/z):263 [M-Cl]⁺
(3) To a four-neck flask that had been sufficiently substituted with nitrogen were put 1237 g of a crude product of 3-{benzyl[(2R)-2-chloro-2-phenylethyl]amino}propanenitrile and 3.71 L of toluene. After cooling to -25°C, 2.73 L (2.73 mol) of 1 M sodium hexamethyldisilazide/tetrahydrofuran was added dropwise thereto at -30 to -25°C. After stirring at -28°C for 15 minutes, 148 mL of water was added thereto. 2.82 L of water was added thereto at 0°C, and the organic layer (the upper layer) was collected by separation. The organic layer collected by separation was washed with brine (445 g of salt/2.23 L of water). The obtained organic layer was concentrated under reduced pressure to obtain 764 g of a mixture (88:12) of (3R,4S)-1-benzyl-4-phenylpyrrolidine-3-carbonitrile and (3S,4S)-1-benzyl-4-phenylpyrrolidine-3-carbonitrile as a crude product (oily substance).
   In this regard, (3R,4S)-1-benzyl-4-phenylpyrrolidine-3-carbonitrile and (3S,4S)-1-benzyl-4-phenylpyrrolidine-3-carbonitrile were separated and purified by silica gel column chromatography, and each of the structures was confirmed.
   (3R,4S)-1-Benzyl-4-phenylpyrrolidine-3-carbonitrile
   ¹H NMR (CDCl₃, 400 MHz) δ (ppm) 7.37-7.24 (m, 10H), 3.74 (d, J=12.8, 1H), 3.69 (d, J=12.8, 1H), 3.14-3.03 (m, 3H), 2.93 (dd, J=8.8, 6.9, 1H), 2.81 (m, 1H).
   MS (API-ES (Pos.)) (m/z):263[M+1]⁺
   (3S,4S)-1-Benzyl-4-phenylpyrrolidine-3-carbonitrile
   ¹H NMR (CDCl₃, 400 MHz) δ (ppm) 7.38-7.25 (m, 10H), 3.76 (s, 2H), 3.66 (dd, J=8.6, 7.7, 1H), 3.47 (ddd, J=9.6, 7.5, 6.2, 1H), 3.22-3.12 (m, 2H), 2.98 (dd, J=9.5, 6.2, 1H), 2.89 (dd, J=9.5, 8.0, 1H).
   MS (API-ES (Pos.)) (m/z):263[M+1]⁺
(4) To a four-neck flask that had been sufficiently substituted with nitrogen were put 762 g of a mixture of (3R,4S)-1-benzyl-4-phenylpyrrolidine-3-carbonitrile and (3S,4S)-1-benzyl-4-phenylpyrrolidine-3-carbonitrile and 3.25 L of ethanol. An aqueous sodium hydroxide solution (310 g of sodium hydroxide/ 650 mL of water) was added thereto, followed by heating and refluxing for 7 hours. After cooling to 11 °C, the reaction liquid was adjusted to pH 4.0 with 710 mL of concentrated hydrochloric acid. 6.50 L of water was added thereto, followed by adjusting to pH 4 with 22 mL of a 24% aqueous sodium hydroxide solution. After stirring at 6°C for 1 hour, the precipitate was filtered, and washed sequentially with an aqueous ethanol solution (650 mL of ethanol/1.3 L of water) and 1.95 L of toluene. The wet crystals thus obtained was dried under reduced pressure to obtain 589 g of (3R,4S)-1-benzyl-4-phenylpyrrolidine-3-carboxylic acid (crystals, yield of 84.5% from (R)-(+)-styrene oxide).
   ¹H NMR (NaOD+D₂O, 400 MHz) δ (ppm) 7.16-7.02 (m, 10H), 3.48 (d, J=12.4, 1H), 3.37 (d, J=12.8, 1H), 3.30(q, J=8.4, 1H), 2.87 (t, J=7.6, 1H), 2.77-2.66 (m, 3H), 2.39 (t, J=8.8, 1H)
   MS (API-ES (Pos.)) (m/z):282[M+1]⁺
(5) To a four-neck flask that had been sufficiently substituted with nitrogen were put 587 g (2.09 mol) of (3R,4S)-1-benzyl-4-phenylpyrrolidine-3-carboxylic acid and 2.94 L of toluene. At 1 to 14°C, 2.41 kg (8.36 mol) of sodium bis(2-methoxyethoxy)aluminum hydride (registered trademark Red-Al™)/a toluene solution (ca 70%) were added dropwise thereto, followed by stirring at 20 to 30°C for 3 hours. On the other hand, to a four-neck flask to which an aqueous sodium hydroxide solution (1.04 kg of sodium hydroxide/4.11 L of water) had been put was added dropwise a reaction liquid at 10 to 20°C, followed by washing with 147 mL of toluene. At 18 to 19°C, 1.76 L of water was added thereto, and the organic layer (the upper layer) was collected by separation. The organic layer collected by separation was washed sequentially with 1.76 L of water and brine (352 g of salt/1.76 L of water). The obtained organic layer was concentrated under reduced pressure to obtain 524 g of [(3R,4S)-1-benzyl-4-phenylpyrrolidin-3-yl]methanol (crystal, yield 93.9%, 99.6%ee).
   ¹H NMR (CDCl₃, 400 MHz) δ (ppm) 7.32-7.17 (m, 10H), 3.72 (dd, J=10.4, 4.0, 1H), 3.65-3.61 (m, 3H), 3.24-3.17 (m, 2H), 2.85-2.76 (m, 2H), 2.48-2.37 (m, 2H).
   MS (API-ES (Pos.)) (m/z):268[M+1]⁺

### Example 2

To a four-neck flask that had been sufficiently substituted with nitrogen were put 2.00 g (8.80 mol) of (R)-N-benzyl-2-phenylglycinol, 8.40 g (158 mmol) of acrylonitrile, and 528 mg (8.80 mmol) of acetic acid, followed by heating and refluxing for 41 hours. After cooling to room temperature, the reaction liquid was concentrated under reduced pressure. The obtained residue was purified by silica gel column chromatography (eluent solvent ethyl acetate:n-heptane=1:10→1:4) to obtain 2.18 g (yield 88.4%) of an oily substance. To a three-neck flask that had been sufficiently substituted with nitrogen were added 988 mg (3.57 mmol) of the obtained oily substance and 4.9 ml of ethyl acetate. After cooling to 2°C, 499 mg (4.93 mmol) of triethylamine was added thereto. At 2 to 17°C, 484 mg (4.28 mmol) of methanesulfonyl chloride was added thereto, followed by stirring at 25°C for 3.5 hours. After cooling to 4°C, 3 ml of water was added thereto at 4 to 10°C, and the organic layer (the upper layer) was collected by separation. The organic layer collected by separation was washed sequentially with an aqueous sodium hydrogen carbonate solution (150 mg of sodium hydrogen carbonate/3 mL of water) and brine (600 mg of salt/3 mL of water). The obtained organic layer was concentrated under reduced pressure to obtain 1.06 g of 3-{benzyl[(2S)-2-chloro-2-phenylethyl]amino}propanenitrile (oily substance, yield 101%).
In this regard, the obtained 3-{benzyl[(2S)-2-chloro-2-phenylethyl]amino}propanenitrile was purified by silica gel column chromatography, and the structure was confirmed.
¹H NMR (CDCl₃, 400 MHz) δ (ppm) 7.36-7.23 (m, 10H), 4.80 (t, J=6.8, 1H), 3.74 (d, J=13.6, 1H), 3.68 (d, J=13.6, 1H), 3.14 (dd, J=14.2, 6.8, 2H), 2.85 (t, J=6.8, 2H), 2.31-2.25 (m, 2H).
MS (API-ES (Pos.)) (m/z): 263 [M-Cl]⁺

### Industrial Applicability

As described above, according to the production method of the present invention, optically active pyrrolidine compounds (7) and (8), and an intermediate thereof, in particular, an optically active chloro compound (3) that is a key intermediate can be produced efficiently. The optically active pyrrolidine compounds (7) and (8) obtained by the production method of the present invention are useful as a production intermediate of a pharmaceutical, in particular, a calcium-sensing receptor agonist.

## Claims

1. A method for producing a compound represented by the formula (3) 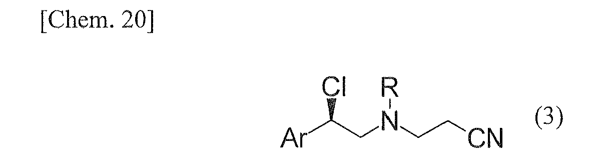 [wherein Ar and R have the same meanings as in the formula (1)] or a salt thereof, which comprises reacting
a compound represented by the formula (1) 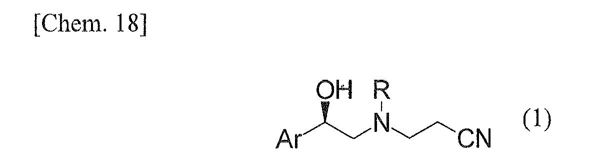 [wherein Ar represents aryl which may be substituted and R represents lower alkyl or lower alkylene-aryl, provided that the aryl in R may be substituted] or a salt thereof, or
a compound represented by the formula (2) 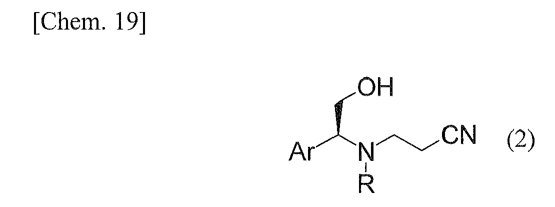 [wherein Ar and R have the same meanings as in the formula (1)] or a salt thereof, or
a mixture of the compound represented by the formula (1) or a salt thereof and the compound represented by the formula (2) or a salt thereof,
with a compound represented by lower allcyl-S(O)₂Cl, in the presence of a base.

2. The production method as described in claim 1,
wherein the compound represented by the formula (1) or a salt thereof or the compound represented by the formula (2) or a salt thereof, or a mixture of the compound represented by the formula (1) or a salt thereof and the compound represented by the formula (2) or a salt thereof, which is produced by reacting a compound represented by the formula (4) 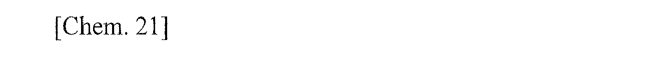 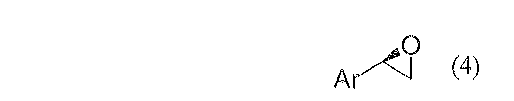 [wherein Ar has the same meaning as in claim 1] with a compound represented by the formula (5) 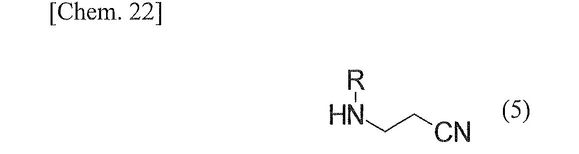 [wherein R has the same meaning as in claim 1] or a salt thereof,
is used as a starting material.

3. A method for producing a compound represented by the formula (6) or a salt thereof, which comprises subjecting the compound represented by the formula (3) or a salt thereof produced by the production method as described in claim 1 or 2 to cyclization using a base: 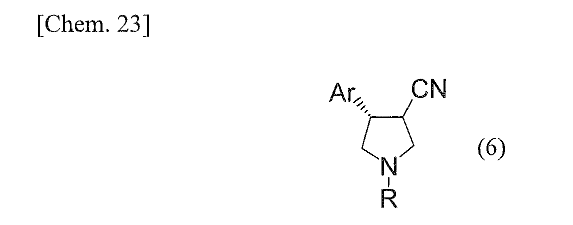 [wherein Ar and R have the same meanings as in claim 1].

4. A method for producing a compound represented by the formula (7) or a salt thereof, which comprises subjecting the compound represented by the formula (6) or a salt thereof produced by the production method as described in claim 3 to hydrolysis using a base: 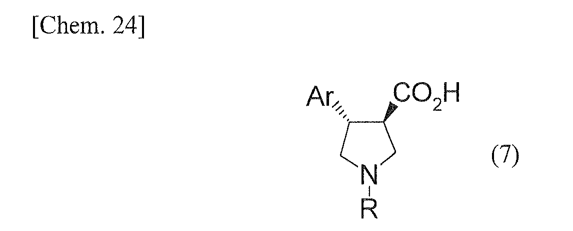 [wherein Ar and R have the same meanings as in claim 1].

5. A method for producing a compound represented by the formula (8) or a salt thereof, which comprises subjecting the compound represented by the formula (7) or a salt thereof produced by the production method as described in claim 4 to reduction: 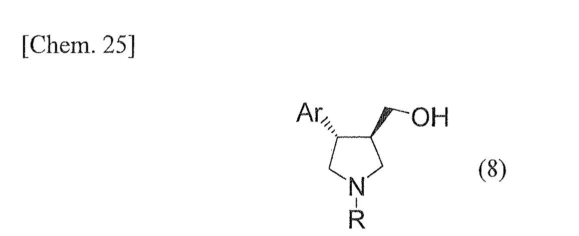 [wherein Ar and R have the same meanings as in claim 1].

6. The production method as described in claim 1, wherein Ar is phenyl which may be substituted.

7. The production method as described in claim 1, wherein R is -CH₂-(phenyl which may be substituted).

8. The production method as described in claim 2, wherein, in the step of reacting the compound represented by the formula (4) or a salt thereof with the compound represented by the formula (5) or a salt thereof in claim 2, the compound represented by the formula (4) or a salt thereof and the compound represented by the formula (5) or a salt thereof are reacted in the presence of phenol.
